# EUROPEAN PATENT SPECIFICATION

(11) **EP 1 981 392 B1**
(45) Date of publication and mention of the grant of the patent: **17.03.2010**
(21) Application number: 07709178.3
(22) Date of filing: 25.01.2007
(51) Int. Cl.: A61B 1/005, A61B 1/31, A61B 1/01, G02B 23/24

(54) **APPARATUS SUITED FOR USE IN A SPACE WHICH IS DIFFICULTLY ACCESSIBLE**
ZUR VERWENDUNG AN SCHWER ZUGÄNGLICHEN ORTEN GEEIGNETE VORRICHTUNG
DISPOSITIF CONÇU POUR ÊTRE UTILISÉ DANS UN ESPACE DIFFICILEMENT ACCESSIBLE

(30) Priority: 30.01.2006 NL 1031031
(43) Date of publication of application: 22.10.2008
(73) Proprietor: Technische Universiteit Delft, 2628 CN Delft (NL)
(72) Inventor: BREEDVELD, Paulus, NL-2806 DK Gouda (NL)
(74) Representative: Van Breda, Jacobus
(86) International application number: PCT/NL2007/050030
(87) International publication number: WO 2007/086743

(56) References cited:
- DE-A1- 4 132 281
- US-A- 4 561 427
- US-A- 5 571 114
- US-A1- 2003 088 152
- US-A1- 2005 154 278

## Description

The invention relates to an apparatus suitable for use in a space that is not easily accessible, comprising a distally provided head, which can be equipped with an inspection organ and/or intervention organ, and which is designed to be introduced into a cavitous body, a proximal control member remaining permanently outside the body, and a connecting body extending between the control member and the distal head, wherein the connecting body is designed for being at least partly introduced into the cavitous body.

Such an apparatus is useful for examining an industrial installation, for example, the steam pipes of a reactor. An example of such an apparatus equipped with an inspection organ is known from the international patent application WO 00/54043 A1.

Such an apparatus is also useful, albeit in a greatly miniaturised form, as instrument for medical examination. Such an instrument is described in the international patent application WO 2006/019291 A1.

Both the apparatus to be used in industry and the apparatus for medical application may, complementary to, or instead of the inspection organ, be equipped with an intervention organ. Where a medically applicable apparatus is concerned, such an intervention organ serves, for example, for performing a surgical operation or the very exact introduction of medication. Another example of a medical intervention organ is the generally known gripping instrument for performing a biopsy. With an industrially applied apparatus, the intervention organ may be used for repair work.

In the case of industrial inspection systems according to the prior art, the propulsion of the head of the apparatus in the cavitous body to be examined or treated is provided by electromagnetic driving means. With medical (inspection) systems, such as the usual flexible endoscopes, gastroscopes or colonoscopes the head has to be displaced by proximally pushing the connecting body forward instead the head being provided with a drive element. A drawback of this is that the head may catch behind protuberances inside the patient, which may cause the connecting body of the scope to buckle as a result of the pushing force. This may be painful for the patient and may cause internal injury.

From the American patent specification US-A-5,571,114 an apparatus according to the preamble is known, wherein the head is provided with at least one rotatable guide element for at least one coupling element, a first end of which is coupled to a permanently fixed point and a second end is position-variable, and wherein the first and the second ends of the coupling element are located at the side of the control member.

In the apparatus known from the American patent specification US-A-5,571,114, said coupling element is provided in the form of a chain or strip reciprocally moving at the outside of and along the connecting body that extends between head and control member, which connecting body may contain, for example, a fibre glass bank.

By driving the rotatable guide element located near the head of the known apparatus, the connecting body of the known apparatus that is coupled to an end of the chain or strip next to it is moved inside the body to be examined. In order to drive the guide element, a drive shaft extending in the longitudinal direction of the connecting body is required.

A drawback of the known apparatus is the relative complexity of the construction which is maintenance-prone and, owing to the use of the broad chain or strip, it is flexurally relatively stiff in the transverse direction. As a result, the known apparatus is not particularly suitable for practical applications, in particular where medical application is concerned.

American patent specification US-A-5,571,114 discloses an apparatus according to the preamble of claim 1.

The object of the invention is to provide an apparatus that can be moved in a reliable manner through the inside of the cavitous body to be inspected or treated.

A further object of the invention is to avoid using the standard electromagnetic drive in the head.

Still another object of the invention is to provide an apparatus that is simple to clean and, if necessary, disinfect.

Still another object of the invention is to provide an apparatus that can be readily miniaturised.

Still another object of the invention is to provide an apparatus that is well suited to be used in aggressive environments caused, for example, by high temperatures, strong chemicals or radioactivity. Underwater applications involving difficult conditions (high water pressure), form an area of application for which the apparatus according to the invention intends to find a solution.

To this end the apparatus according to the invention is characterised by one or several of the appended claims.

In a first aspect of the invention, the apparatus comprises a connecting body that is not compressible and surrounds the at least one coupling element, acting as guide, in that the at least one rotatable guide element is designed to provide traction along a wall of the body, and that the control member is adjustable in relation to the permanently fixed point, in order to displace the at least one coupling element in the longitudinal direction inside the connecting body in such a manner, that the rotatable guide element providing the traction begins to rotate characterised in that the connecting body (4) connects the control member (5) to the distal head (1).

This enables the rotatable guide element to move the head along the wall of the body without the risk that the movement of the control member will cause the connecting body to buckle.

It is also an advantage that the traction-providing bypass organ does not comprise electromechanical means for realising the displacement, so that the apparatus can be manufactured at low costs and is suitable for simple cleaning and disinfection. It is also extremely simple to miniaturise the apparatus according to the invention. Due to the low manufacturing costs, the apparatus is moreover particularly suitable to be embodied as disposable, which is especially desirable in the case of medical applications.

In order to be able to follow bends in the cavitous body to be inspected or treated, it is further desirable for the connecting body to be bendable around the longitudinal axis.

It is also useful that the second end of the at least one coupling element has tensile strain-bearing capacity. This ensures that an accurate relation is maintained between the displacement of the control member and the corresponding rotation of the rotatable guide element.

The tensile strain-bearing capacity referred to realised in different ways, for example, by suspending a weight from the second end, as shown in the figure legend included with this application. However, other variants are sometimes also advantageous, for example, a mechanical coupling between the first end and the second end of the coupling element, which maintains a fixed relation between both positions. A suitable example for this is a gearbox. A simple spring load at the second end may also suffice.

The bypass organ may be selected from various embodiments. However, it is preferred for the at least one to be a predetermined choice from the group comprising a pulley, a track chain, a propulsion organ as described in the international patent application PCT/NL05/000564.

It is also possible to use various bypass elements that are different from each other.

In a first preferred embodiment, the apparatus is **characterised in that** a series of guide elements is provided arranged in the form of a star around a longitudinal axis of the apparatus.

A second preferred embodiment of the apparatus according to the invention is **characterised, in that** a succession of guide elements is provided along the longitudinal axis of the apparatus.

Another aspect of the invention relates to the embodiment of the apparatus that is **characterised in that** there are at least two rotatable guide elements, of which at least one guide element is placed on its own hinged arm.

This makes it possible in a simple manner to use the apparatus with cavity-like bodies of varying diameters.

It is furthermore desirable for at least one of the arms of the guide element to have a preferential position such that the guide element incline away from each other. This ensures a continuous contact of the guide element with the wall of the cavitous body to be inspected or treated.

Hereinafter the invention will be further elucidated by way of a schematic exemplary embodiment as shown in the drawing illustrating the working principle of the apparatus according to the invention.

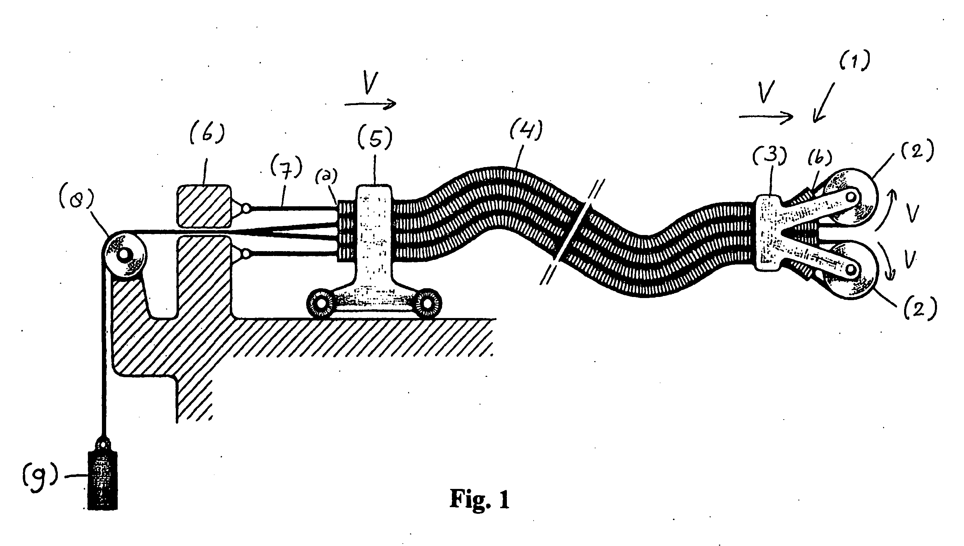

In the drawing, Fig. 1 schematically shows an apparatus, with the omission of some of the components that are of little importance with respect to explaining the invention.

Fig. 1 shows that the apparatus possesses a distally placed head 1. This head 1 may be equipped with an inspection organ and/or intervention organ (not shown) and is designed to be introduced into a cavitous body to be inspected or treated. In industrial applications this cavitous body may, for example, be the inside of steam pipes of a boiler or reactor. In the case of medical applications this may, for example, be an intestine.

At the proximal side and intended to remain permanently outside of the body to be inspected or treated, a control member 5 is provided. A connecting body 4 extends between the control member 5 and the head 1, connecting the control member 5 and the head 1. Said connecting body 4 is also designed for being at least partly introduced into the cavitous body to be inspected or treated.

In accordance with the invention, the connecting body 4 is not compressible and serves also as housing for the at least one coupling element 7 extending through the connecting body 4. As such the connecting body 4 acts as guide for the at least one coupling element.

In the case illustrated, two coupling elements 7 are shown in the form of cables running through the connecting body 4 from outside the control member 5 to the head 1, and from the head 1 back to outside the operating member 5.

In order to facilitate the reciprocal movement of the cables 7, the head is provided with at least one, in the illustrated case with two, guide element 2. These guide elements 2 are designed to provide traction along a wall of the body and in order to achieve this traction, the cables 7 are at a first end coupled with a permanently fixed point 6, while a second end is position-variable. In the case shown, this second end is provided with a weight 9 and the cables 7 run over a pulley 8, the gravitational force keeping the cables 7 under tension strain. As has been explained above, it is also possible to apply other methods in order to produce this tension strain.

Fig. 1 shows that both the first and the second end of the cables 7 are located at the side of the control member 5, outside the connecting body 4.

It is further of importance that in order to displace the cables 7 running through the connecting body 4 in the longitudinal direction, the control member 5 can be adjusted in relation to the permanently fixed point 6 by hand or by means of a motor drive. As a consequence of this displacement, the pulleys 2 begin to rotate and, as these pulleys are coupled with the wall of the cavitous body to be inspected or treated, they ensure that at the head side 1 a propelling force is applied to the apparatus.

The workings of the construction may be illustrated as follows.

By displacing the control member 5 in the direction of arrow V the upper and lower cable 7 is pulled out of the connecting body 4. Owing to the connecting body 4 not being compressible, the upper and lower cable 7 is pulled inward at the side of the head 1. Because the cables 7 are guided around the pulleys 2, said pulleys 2 are caused to rotate at a rotational speed that corresponds with the displacement speed of the control member 5. In the absence of slip between the pulleys 2 and the wall of the cavitous body to be inspected or treated, the head 1 will correspondingly move at the same speed as the control member 5, so that pushing forces on the connecting body 4 are avoided and there is no risk of the connecting body 4 buckling.

Although Fig. 1 shows that two pulleys 2 are used, this number may be increased as desired up to a series of pulleys arranged, for example, in the shape of a star around the longitudinal axis of the connecting body 4.

It is also possible to arrange along the longitudinal axis of the connecting body 4 several pulleys 2 in succession.

It will be further obvious to the person skilled in the art that the connecting body 4 is preferably bendable around the longitudinal axis, which, owing to the construction of the apparatus according to the invention involves no risk of the connecting body 4 buckling. However, this flexibility does make it possible to easily follow curves in the cavitous body to be inspected or treated. Although Fig. 1 shows a pulley being used as rotatable guide element 2, it is also possible to use for this purpose a track chain or a propulsion organ as described in the international patent application PCT/NL05/000564.

It is further observed that Fig. 1 shows that the guide elements 2 used are each mounted on a rigid arm. Within the scope of the invention it is also useful and even advantageous to embody at least one of these arms, and preferably both arms, to be hinged in such a manner that said arms have a preferential position in which the guide elements 2 incline away from each other. This renders it easy to follow the diameter variations of the cavitous body to be inspected.

The research culminating in the present invention was made possible by a grant from the Royal Netherlands Academy of Arts and Sciences.

## Claims

1. An apparatus suitable for use in a space that is not easily accessible, comprising a distally provided head (1), which can be equipped with an inspection organ and/or intervention organ, and which is designed to be introduced into a cavitous body, a proximal control member (5) remaining permanently outside the body, and a connecting body (4) extending between the control member (5) and the distal head (1), wherein the connecting body (4) is designed for being at least partly introduced into the body, and wherein the head (1) is provided with at least one rotatable guide element (2) for at least one coupling element (7), a first end of which at least one coupling element (7) is coupled to a permanently fixed point (6) and a second end is position-variable, and wherein the first and the second ends of the coupling element (7) are located at the side of the control member (5), the connecting body (4) is not compressible and surrounds the at least one coupling element (7), acting as guide, in that the at least one guide element (2) is designed to provide traction along a wall of the body, and that the control member (5) is adjustable in relation to the permanently fixed point (6), in order to displace the at least one coupling element. (7) in the longitudinal direction inside the connecting body (4) in such a manner, that the guide element (2) providing the traction begins to rotate, **characterised in that** the connecting body (4) connects the control member (5) to the distal head (1).

2. An apparatus according to claim 1, **characterised in that** the first and the second end of the coupling element (7) are located outside the connecting body (4).

3. An apparatus according to claim 1 or 2, **characterised in that** the connecting body is bendable around the longitudinal axis.

4. An apparatus according to one of the claims 1-3, **characterised in that** the second end of the at least one coupling element (7) is under tensile strain.

5. An apparatus according to one of the claims 1-4, **characterised in that** the at least one rotatable guide element (2) is a predetermined choice from the group comprising a pulley, a track chain, a propulsion organ.

6. An apparatus according to one of the claims 1-5, **characterised in that** that a series of rotatable guide elements (2) is provided arranged in the form of a star around a longitudinal axis of the apparatus.

7. An apparatus according to one of the claims 1-6, **characterised in that** a succession of rotatable guide elements (2) is provided along the longitudinal axis of the apparatus.

8. An apparatus according to one of the claims 1-7, **characterised in that** there are at least two rotatable guide elements (2), of which at least one rotatable guide element is placed on its own hinged arm.

9. An apparatus according to claim 8, **characterised in that** at least one of the arms of the rotatable guide elements has a preferential position such that the rotatable guide elements (2) incline away from each other.

## Patentansprüche

1. Eine Vorrichtung, die zur Verwendung in einem Raum, der nicht ohne weiteres zugänglich ist, geeignet ist und die einen distal vorgesehenen Kopf (1) aufweist, der mit einem Prüforgan und/oder einem Interventionsorgan ausgestattet sein kann und der dahin gehend entworfen ist, in einen einen Hohlraum umfassenden Körper eingebracht zu werden, und wobei ein proximales Steuerbauglied (5) dauerhaft außerhalb des Körpers verbleibt und sich ein Verbindungskörper (4) zwischen dem Steuerbauglied (5) und dem distalen Kopf (1) erstreckt, wobei der Verbindungskörper (4) dahin gehend entworfen ist, zumindest teilweise in den Körper eingebracht zu werden, und wobei der Kopf (1) mit zumindest einem drehbaren Führungselement (2) für zumindest ein Kopplungselement (7) versehen ist, wobei ein erstes Ende des zumindest einen Kopplungselements (7) mit einem dauerhaft feststehenden Punkt (6) gekoppelt ist und ein zweites Ende bezüglich der Position variabel ist, und wobei das erste und das zweite Ende des Kopplungselements (7) an der Seite des Steuerbauglieds (5) angeordnet sind, wobei der Verbindungskörper (4) nicht komprimierbar ist und das zumindest eine Kopplungselement (7) insofern umgibt, wobei er als Führung fungiert, als das zumindest eine Führungselement (2) dahin gehend entworfen ist, eine Zugkraft entlang einer Wand des Körpers zu liefern, und als das Steuerbauglied (5) bezüglich des dauerhaft feststehenden Punktes (6) einstellbar ist, um das zumindest eine Kopplungselement (7) in der Längsrichtung im Inneren des Verbindungskörpers (4) derart zu verschieben, dass das Führungselement (2), das die Zugkraft liefert, sich zu drehen beginnt, **dadurch gekennzeichnet, dass** der Verbindungskörper (4) das Steuerbauglied (5) mit dem distalen Kopf (1) verbindet.

2. Eine Vorrichtung gemäß Anspruch 1, **dadurch gekennzeichnet, dass** das erste und das zweite Ende des Kopplungselements (7) außerhalb des Verbindungskörpers (4) angeordnet sind.

3. Eine Vorrichtung gemäß Anspruch 1 oder 2, **dadurch gekennzeichnet, dass** der Verbindungskörper um die Längsachse herum biegbar ist.

4. Eine Vorrichtung gemäß einem der Ansprüche 1 bis 3, **dadurch gekennzeichnet, dass** das zweite Ende des zumindest einen Kopplungselements (7) unter Zugbelastung steht.

5. Eine Vorrichtung gemäß einem der Ansprüche 1 bis 4, **dadurch gekennzeichnet, dass** das zumindest eine drehbare Führungselement (2) eine vorbestimmte Auswahl aus der Gruppe ist, die eine Riemenscheibe, eine Laufkette, ein Antriebsorgan umfasst.

6. Eine Vorrichtung gemäß einem der Ansprüche 1 bis 5, **dadurch gekennzeichnet, dass** eine Serie von drehbaren Führungselementen (2) in Form eines Sterns um eine Längsachse der Vorrichtung herum vorgesehen ist.

7. Eine Vorrichtung gemäß einem der Ansprüche 1 bis 6, **dadurch gekennzeichnet, dass** eine Abfolge von drehbaren Führungselementen (2) entlang der Längsachse der Vorrichtung vorgesehen ist.

8. Eine Vorrichtung gemäß einem der Ansprüche 1 bis 7, **dadurch gekennzeichnet, dass** es zumindest zwei drehbare Führungselemente (2) gibt, von denen zumindest ein drehbares Führungselement an seinem eigenen angelenkten Arm platziert ist.

9. Eine Vorrichtung gemäß Anspruch 8, **dadurch gekennzeichnet, dass** zumindest einer der Arme der drehbaren Führungselemente eine Vorzugsposition aufweist, so dass sich die drehbaren Führungselemente (2) voneinander weg neigen.

## Revendications

1. Appareil approprié pour une utilisation dans un espace qui n'est pas facilement accessible, comprenant une tête montée de manière distale (1), qui peut être équipée d'un organe d'inspection et/ou d'un organe d'intervention, et qui est conçue pour être introduite dans un corps de cavité, un élément de commande proximal (5) restant de manière permanente à l'extérieur du corps, et un corps de liaison (4) s'étendant entre l'élément de commande (5) et la tête distale (1), où le corps de liaison (4) est conçu pour être au moins partiellement introduit dans le corps, et où la tête (1) est munie d'au moins un élément de guidage rotatif (2) pour au moins un élément de couplage (7), à une première extrémité duquel au moins un élément de couplage (7) est couplé à un point fixé de manière permanente (6) et une deuxième extrémité est à position variable, et où les première et deuxième extrémités de l'élément de couplage (7) sont situées du côté de l'élément de commande (5), le corps de liaison (4) n'étant pas compressible et entourant l'au moins un élément de couplage (7), agissant en tant que guide, l'au moins un élément de guidage (2) étant conçu pour fournir une traction le long d'une paroi du corps, et l'élément de commande (5) étant ajustable par rapport au point fixé de manière permanente (6), afin de déplacer l'au moins un élément de couplage (7) dans la direction longitudinale à l'intérieur du corps de liaison (4) d'une manière telle que l'élément de guidage (2) fournissant la traction commence à tourner, **caractérisé en ce que** le corps de liaison (4) relie l'élément de commande (5) à la tête distale (1).

2. Appareil selon la revendication 1, **caractérisé en ce que** la première extrémité et la deuxième extrémité de l'élément de couplage (7) sont situées à l'extérieur du corps de liaison (4).

3. Appareil selon la revendication 1 ou 2, **caractérisé en ce que** le corps de liaison peut être courbé autour de l'axe longitudinal.

4. Appareil selon l'une quelconque des revendications 1 à 3, **caractérisé en ce que** la deuxième extrémité de l'au moins un élément de couplage (7) est soumise à un effort de traction.

5. Appareil selon l'une quelconque des revendications 1 à 4, **caractérisé en ce que** l'au moins élément de guidage rotatif (2) est un choix prédéterminé parmi le groupe comprenant une poulie, une chaîne de guidage, un organe de propulsion.

6. Appareil selon l'une quelconque des revendications 1 à 5, **caractérisé en ce qu'**une série d'éléments de guidage rotatifs (2) est prévue de manière agencée sous la forme d'une étoile autour d'un axe longitudinal de l'appareil.

7. Appareil selon l'une quelconque des revendications 1 à 6, **caractérisé en ce qu'**une succession d'éléments de guidage rotatifs (2) est prévue le long de l'axe longitudinal de l'appareil.

8. Appareil selon l'une quelconque des revendications 1 à 7, **caractérisé en ce qu'**il existe au moins deux éléments de guidage rotatifs (2), parmi lesquels au moins un élément de guidage rotatif est placé sur son propre bras articulé.

9. Appareil selon la revendication 8 **caractérisée en ce qu'**au moins un des bras des éléments de guidage rotatifs possède une position préférentielle de sorte que les éléments de guidage rotatifs (2) s'inclinent à distance l'un de l'autre.
